Europäisches Patentamt

European Patent Office

Office européen des brevets

(19)

(11) Numéro de publication: **O 003 460**
**B1**

(12) **FASCICULE DE BREVET EUROPEEN**

(45) Date de publication du fascicule de brevet: **29.04.81**

(21) Numéro de dépôt: **79400047.1**

(22) Date de dépôt: **24.01.79**

(51) Int. Cl.³: **C 07 C 91/10, C 07 C 89/00**
**//C07C79/18**

(54) **Préparation du tris (hydroxyméthyl) aminométhane.**

(30) Priorité: **25.01.78 FR 7801965**

(43) Date de publication de la demande:
**08.08.79 Bulletin 79/16**

(45) Mention de la délivrance du brevet:
**29.04.81 Bulletin 81/17**

(84) Etats Contractants Désignés:
**BE CH DE FR GB IT LU NL SE**

(56) Documents cités:
**GB - A - 818 764**
**US - A - 3 564 062**

(73) Titulaire: **SOCIETE CHIMIQUE DE LA GRANDE PAROISSE AZOTE ET PRODUITS CHIMIQUES**
**8, rue Cognacq-Jay**
**F-75007 Paris (FR)**

(72) Inventeur: **Bourguignon, Jean**
**7, Clos du Manoir**
**F-76230 Isneauville (FR)**
Inventeur: **Sion, Marcel-Xavier**
**450, Domaine du Chateau Lewarde**
**F-59287 Lewarde (FR)**
Inventeur: **Moreau, Michel**
**47, rue de Cauville**
**F-76100 Rouen (FR)**

(74) Mandataire: **Bouton Neuvy, Liliane et al,**
**L'Air liquide, Société Anonyme pour L'Etude et L'Exploitation des Procédés Georges Claude 75, Quai d'Orsay**
**F-75321 Paris Cedex 07 (FR)**

Courier Press, Leamington Spa, England.

## Préparation du tris (hydroxyméthyl) aminométhane

La présente invention concerne la préparation du tris (hydroxyméthyl) aminométhane sous forme cristalline.

La préparation du nitroalcool par action du formaldéhyde sur le nitrométhane en milieu basique est connue en soi. L'agent basique est utilisé en proportions molaires ou en quantités catalytiques. les bases sont généralement les hydroxydes alcalins ou alcalino-terreux, les résines échangeuses d'ion basique ou les amines tertaires. Tous les procédés sont mis en oeuvre en milieu aqueux ou hydroalcoolique. Après hydroxyméthylation et élimination de la base, la réduction est réalisée sous pression d'hydrogène, parfois en présence de dioxyde de carbone pour diminuer les réactions secondaires, en présence d'un catalyseur d'hydrogénation; puis suivent des opérations de concentration et de cristallisation nécessitant le recyclage des solvants de recristallisation.

Selon le brevet britannique 818.764, l'hydroxyméthylation est réalisée par le formaldéhyde en solution aqueuse en présence de chaux. Après neutralisation par l'acide sulfurique, le milieu réactionnel doit être dilué par du méthanol, le pH ajusté à 6,8 — 7 et le sulfate de calcium séparé par filtration, après précipitation de celui-ci.

Il a été trouvé un procédé qui, par le double choix du catalyseur et de l'acide, supprime l'opération de filtration avant hydrogénation du nitroalcool, sans aucune conséquence défavorable pour celle-ci. Selon le procédé mis au point on travaille en milieu exempt d'eau dont la présence est préjudiciable lors de l'hydrogénation du nitroalcool. A la fin de la réduction par hydrogénation par simple refroidissement du milieu réactionnel on obtient directement une quantité importante de tris (hydroxyméthyl) aminométhane cristallisé de pureté excellente 99,5%, avec un rendement de l'ordre de 80%, le rendement total étant supérieur à 90%.

Selon l'invention le tris (hydroxyméthyl) aminométhane est préparé en milieu alcoolique concentré par action du polyoxyméthylène sur le nitrométhane en présence d'une quantité catalytique d'un agent alcalin solide et de chlorure de méthylène; suivi d'une acidification et d'une réduction par l'hydrogène en milieu alcoolique contenant du chlorure de méthylène.

Le nitrométhane et le formaldéhyde sous forme solide sont mis en réaction dans un rapport molaire de l'ordre de 1/3 en milieu alcoolique concentré constitué par un mélange de 2 à 5% de chlorure méthylène dans le méthanol, en présence de 1 à 10 milliéquivalents par mole de nitrométhane d'un catalyseur basique sous forme solide, choisi parmi la soude ou la potasse, sous agitation pendant une durée comprise entre 3 et 36 heures à température inférieure à 50°C.

Le produit formé dans la phase d'hydroxyméthylation est acidifié par un acide concentré à un pH compris entre 2,7 et 7, de préférence entre 4,5 et 5,5. Le nitroalcool ainsi obtenu est dilué dans du méthanol contenant 2 à 5% de chlorure de méthylène, de préférence 2,5%; il est ensuite hydrogéné par de l'hydrogène à l'exclusion de tout autre constituant gazeux sous une pression d'hydrogène comprise entre 30 et 60 bars, en présence de nickel de Raney. La quantité de catalyseur d'hydrogénation est choisie de manière telle qu'elle représente 5 à 25% en poids du nitroalcool à réduire.

Après hydrogénation le milieu réactionnel est filtré pour éliminer le catalyseur, et il est refroidi jusqu'à 0°C. Le rendement en premier jet de tris (hydroxyméthyl) aminométhane de grande pureté, séparé directement par refroidissement du milieu réactionnel, atteint 60 — 65%. Par concentration des eaux-mères jusqu'à un résidu huileux repris par un mélange à 30% de méthanol dans le chlorure de méthylène on isole un deuxième jet de tris (hydroxyméthyl) aminométhane de pureté identique au premier jet, portant le rendement total à 75 — 80%. Une troisième cristallisation permet de récupérer un dernier jet de qualité acceptable, avec un rendement tel que le rendement total est supérieur à 90%.

Il est donné ci-après un exemple qui illustre l'invention à titre non limitatif.

### Exemple

A 0,331 mole de nitrométhane dans 35 ml de méthanol contenant 1 ml de chlorure de méthylène, on ajoute sous agitation de la soude en pastilles en quantité telle que le pH soit maintenu entre 8 et 9 du polyoxyméthylène correspondant à 0,933 mole de formaldéhyde. Pendant la réaction la température est comprise entre 45 et 55°C, la durée de réaction sous agitation est de 2 heures.

Lorsque tout le formaldéhyde ajouté est consommé par la réaction, on abaisse le pH à 4,5 par addition de l'acide chlorhydrique concentré.

On introduit le nitroalcool ainsi obtenu dilué dans 250 ml de méthanol et 2 ml de chlorure de méthylène dans le réacteur d'hydrogénation. On hydrogène le mélange sur du nickel de Ranay représentant 5% en poids du nitroalcool à réduire, sous une pression de 30 bars à une température de 40°C à 47°C.

Après la fin de l'absorption de l'hydrogéne, on sépare le catalyseur par filtration et on le lave par 90 ml de méthanol à 2% de chlorure de méthylène. Le méthanol de lavage est réuni au produit réactionnel.

On refroidit le produit réactionnel en milieu méthanol pendant deux heures à 0°C. Par cristallisation, on obtient le trishydroxyméthane avec un rendement molaire de 62% d'un produit de fusion 170°C de pureté supérieure à 99,5% déterminée par dosage acidimétrique. La micro-analyse de ce produit est correcte, c'est-à-dire que les pourcentages trouvés en C, H et N ne diffèrent pas de plus de 0,2% en valeur absolue de la valeur théorique. On évapore les eaux-mères de première cristallisation, et l'on reprend le résidu huileux avec 5 ml de méthanol et 10 ml de chlorure de méthylène. Après refroidissement, on obtient des cristaux qui sont lavés par le mélange froid méthanol-chlorure de méthylène, puis au méthanol; et le rendement total des première et deuxième cristallisations est de 77,6% (hydroxyméthyl) aminométhane de point de fusion 169°C, de pureté 98% par acidimétrie. La micro-analyse est toujours correcte. Une troisième cristallisation permet de récupérer encore une certaine quantité de tris (hydroxyméthyl) aminométhane de pureté voisine de 93% déterminée par acidimétrie, d'un point de fusion de 165°C, et de porter le rendement total au-delà de 90%.

## Revendications

1. Procédé de préparation du tris (hydroxyméthyl) aminométhane pur sous forme cristalline séparé directement par refroidissement du milieu réactionnel, par hydroxyméthylation puis hydrogénation, caractérisé en ce que le nitrométhane et le formaldéhyde, sous forme solide ou polyoxyméthylène, sont mis en réaction dans un rapport molaire de l'ordre de 1/3 en milieu alcoolique concentré constitué par un mélange de 2 à 5% de chlorure de méthylène dans le méthanol, en présence de 1 à 10 milliéquivalents par mole de nitrométhane d'un catalyseur basique sous forme solide, choisi parmi la soude ou la potasse, sous agitation pendant une durée comprise entre 3 et 36 heures à température inférieure à 50°C, le produit obtenu est acidifié par l'acide chlorhydrique concentré jusqu'à pH compris entre 4,5 et 5,5, le nitroalcool est dilué dans du méthanol à 2 à 5% de chlorure de méthylène puis hydrogéné catalytiquement sur nickel de Raney, sous une pression comprise entre 30 et 60 bars et à température entre 40 et 47°C.

2. Procédé de préparation du tris (hydroxyméthyl) aminométhane selon la revendication 1, caractérisé en ce que le poids du nickel de Raney est compris entre 5 et 25% par rapport au poids du nitroalcool à réduire.

3. Procédé de préparation du tris (hydroxyméthyl) aminométhane selon la revendication 1, caractérisé en ce que le méthanol de dilution contient 2,5% de chlorure de méthylène.

4. Procédé de préparation du tris (hydroxyméthyl) aminométhane selon la revendication 1, caractérisé en ce que le premier jet de produit final sous forme cristalline est séparé directement par refroidissement du milieu réactionnel exempt du catalyseur d'hydrogénation, puis le second jet, de même pureté que le premier est obtenu après séparation de celui-ci, concentration des eauxmères jusqu'à un résidu huileux repris par un mélange à 30% de méthanol dans le chlorure de méthylène.

## Claims

1. Process for the preparation of pure tris-(hydroxymethyl)-amino methane in crystalline form separated directly from the reaction medium by cooling, by hydroxymethylation and subsequent hydrogenation, characterized in that the nitromethane and the formaldehyde, the latter one in solid form or in the form of polyoxymethylene, are reacted in mol ratio in the order of 1/3 in a concentrated alcoholic medium consisting of a mixture of 2 to 5% of methylene chloride in methanol, in presence of 1 to 10 milliequivalents per mol of nitromethane of a basic catalyst in solid form selected from the group of soda and potash, with agitation during 3 and 36 hours at a temperature below 50°C, the obtained product is acidified by concentrated hydrochlorid acid to obtain a pH between 4,5 and 5,5, the nitroalcohol is diluted by methanol containing 2 to 5% of methylene chloride and then catalytically hydrogenated in presence of Raney-nickel at a pressure between 30 and 60 bar and a temperature between 40 and 47°C.

2. Process for the preparation of tris-(hydroxymethyl)-amino methane according to claim 1, characterized in that the weight of the Raney-nickel is between 5 and 25%, based on the weight of the nitroalcohol to be reduced.

3. Process for the preparation of tris-(hydrodxymethyl)-amino methane according to claim 1, characterized in that the methanol for the dilution contains 2,5% of methylene chloride.

4. Process for the preparation of tris-(hydroxymethyl)-amino methane according to claim 1, characterized in that the first crop of the final product in crystalline form is separated directly by cooling the reaction medium free from hydrogenation catalyst and then the second crop having the same purity as the first one, is obtained after separation of this one by concentration of the mother liquors up to an oily residue and taking up by a mixture of up to 30% of methanol in methylene chloride.

## Patentansprüche

1. Verfahren zur herstellung von reinem Tris-(hydroxymethyl)-aminomethan in kristalliner Form, die sich durch Abkühlen direkt vom Reaktionsmedium abtrennt, durch Hydroxymethylierung und anschließende Hydrierung, dadurch gekennzeichnet, daß das Nitromethan und der Formaldehyd, letzterer in fester Form oder in der Form von Polyoxymethylen, in einem Molverhältnis in der Größenordnung von 1/3 in konzentriertem alkoholischem Medium, das aus einem Gemisch von 2 bis 5% Methylenchlorid in Methanol besteht, in Gegenwart von 1 bis 10 Milliäquivalenten eines basischen Katalysators in fester Form, der unter den Verbindungen Soda und Pottasche ausgewählt ist, je Mol Nitromethan unter Rühren während einer Dauer von 3 bis 36 Stunden bei einer Temperatur unterhalb 50°C miteinander umgesetzt werden, das erhaltene Produkt mit konzentrierter Chlorwasserstoffsäure bis zu einem pH-Wert zwischen 4,5 und 5,5 angesäuert wird, der Nitroalkohol mit Methanol mit einem Gehalt von 2 bis 5% Methylenchlorid verdünnt und dann katalytisch über Raney-Nickel unter einem Druck zwischen 30 und 60 bar und bei einer Temperatur zwischen 40 und 47°C hydriert wird.

2. Verfahren zur Herstellung von Tris-(hydroxymethyl)-aminomethan nach Anspruch 1, dadurch gekennzeichnet, daß das Gewicht des Raney-Nickels zwischen 5 und 25%, bezogen auf das Gewicht des zu reduzierenden Nitroalkohols, liegt.

3. Verfahren zur Herstellung von Tris-(hydroxymethyl)-aminomethan nach Anspruch 1, dadurch gekennzeichnet, daß das Methanol für die Verdünnung 2,5% Methylenchlorid enthält.

4. Verfahren zur Herstellung von Tris-(hydroxymethyl)-aminomethan nach Anspruch 1, dadurch gekennzeichnet, daß der erste Ertrag des Endproduktes in kristalliner Form direkt durch Abkühlung des von Hydrierkatalysator freien Reaktionsmediums abgetrennt wird und sodann der zweite Ertrag von gleicher Reinheit wie der erste nach Abtrennung desselben durch Konzentrierung der Mutterlaugen bis zu einem öligen Rückstand und Wiederaufnehmen mit einem Gemisch von bis zu 30% Methanol in Methylenchlorid erhalten wird.